Europäisches Patentamt

European Patent Office

Office européen des brevets

(19)

(11) Veröffentlichungsnummer: **0 165 476**
A2

(12) # EUROPÄISCHE PATENTANMELDUNG

(21) Anmeldenummer: 85106067.3

(22) Anmeldetag: 17.05.85

(51) Int. Cl.⁴: **C 07 K 15/06**
C 07 K 15/14, C 07 K 3/28
G 01 N 33/68

(30) Priorität: 21.05.84 DE 3418888

(43) Veröffentlichungstag der Anmeldung:
27.12.85 Patentblatt 85/52

(84) Benannte Vertragsstaaten:
AT BE CH DE FR GB IT LI LU NL SE

(71) Anmelder: BEHRINGWERKE AKTIENGESELLSCHAFT
Postfach 1140
D-3550 Marburg/Lahn(DE)

(72) Erfinder: Bohn, Hans, Dr.
Oberer Eichweg 26
D-3550 Marburg 1(DE)

(72) Erfinder: Winckler, Wilhelm
Untere Bergstrasse 21
D-3556 Wenkbach(DE)

(74) Vertreter: Becker, Heinrich Karl Engelbert, Dr. et al,
HOECHST AKTIENGESELLSCHAFT Central Patent
Department P.O. Box 80 03 20
D-6230 Frankfurt am Main 80(DE)

(54) Gewebeprotein PP18, Verfahren zu seiner Gewinnung sowie seine Verwendung.

(57) Es wird das Protein $PP_{18}$, gekennzeichnet durch
  a) eine elektrophoretische Beweglichkeit im Bereich der von $\beta_1$-Globulinen;
  b) einen isoelektrischen Punkt zwischen 5,6 und 6,2;
  c) einen Sedimentationskoeffizienten $s_{20,w}$ von 5,0 ±0,2 S;
  d) ein in einer Ultrazentrifuge bestimmtes Molekulargewicht von 82 300 ± 5 600;
  e) einen Kohlenhydratanteil von 2,3 ± 1,3 g/100 g (Mannose 0,15 ± 0,1, Xylose (?) 0,5 ± 0,5, Galactose 0,5 ± 0,2, Glukose 0,2 ± 0,1, N-Acetyl-Glukosamin 0,7 ± 0,2, N-Acetyl-Neuraminsäure 0,25 ± 0,2, jeweils g/100 g) und eine bestimmte Aminosäurenzusammensetzung sowie ein Verfahren zu seiner Gewinnung und seine Verwendung beschrieben.

EP 0 165 476 A2

BEHRINGWERKE AKTIENGESELLSCHAFT   -1- 84/B 010   0165476
Dr. Ha/Sd.

Gewebeprotein PP18, Verfahren zu seiner Gewinnung sowie seine Verwendung

---

Die Erfindung betrifft ein Gewebeprotein, das als $PP_{18}$ bezeichnet wird, sowie ein Verfahren zu seiner Gewinnung. $PP_{18}$ kann verwendet werden, um Antiseren herzustellen, die dazu dienen können, $PP_{18}$ im Gewebe und in Körperflüssigkeiten nachzuweisen und zu bestimmen, um Erkrankungen bestimmter Organe zu erkennen, als "Marker" einen Krankheitsverlauf zu überwachen oder eine Therapie zu kontrollieren.

Proteine sind zwar im Stand der Technik bekannt, auch Gewebeproteine, jedoch keines mit den nachfolgend für $PP_{18}$ angegebenen Eigenschaften.

Gegenstand der Erfindung ist das Protein $PP_{18}$, gekennzeichnet durch

a) eine elektrophoretische Beweglichkeit im Bereich der von $\beta_1$-Globulinen;

b) einen isoelektrischen Punkt zwischen 5,6 und 6,2;

c) einen Sedimentationskoeffizienten $s_{20,w}$ von 5,0 ± 0,2 S;

d) ein in einer Ultrazentrifuge bestimmtes Molekulargewicht von 82 300 ± 5 600; und

e) einen Kohlenhydratanteil von 2,3 ± 1,3 g/100 g (Mannose 0,15 ± 0,1, Xylose(?) 0,5 ± 0,5, Galactose 0,5 ± 0,2, Glukose 0,2 ± 0,1, N-Acetyl-Glukosamin 0,7 ± 0,2, N-Acetyl-Neuraminsäure 0,25 ± 0,2, jeweils g/100 g).

Die Aminosäurenzusammensetzung von $PP_{18}$ ist in der folgenden Tabelle gegeben:

| Aminosäure | Reste pro 100 Reste | Variations-koeffizient |
|---|---|---|
| Lysin | 5,26 | 0,13 |
| Histidin | 2,20 | 7,71 |
| Arginin | 5,53 | 1,41 |
| Asparaginsäure | 9,26 | 4,20 |
| Threonin | 3,60 | 4,32 |
| Serin | 5,21 | 2,58 |
| Glutaminsäure | 10,37 | 0,82 |
| Prolin | 8,13 | 1,48 |
| Glycin | 7,18 | 0,30 |
| Alanin | 4,97 | 1,00 |
| Cystin 1/2 | 1,78 | 0,79 |
| Valin | 9,05 | 1,09 |
| Methionin | 3,00 | 1,65 |
| Isoleucin | 3,33 | 2,55 |
| Leucin | 11,81 | 0,66 |
| Tyrosin | 2,37 | 4,77 |
| Phenylalanin | 4,21 | 1,51 |
| Tryptophan | 2,70 | 1,84 |

Zur Erläuterung der kennzeichnenden Merkmale des Gewebsproteins sei folgendes ausgeführt:

Die elektrophoretische Beweglichkeit wurde in der Mikromodifikation mit dem Gerät Microzone R 200 von Beckman Instruments auf Zelluloseazetatfolien (Firma Sartorius) unter Verwendung von Natriumdiethylbarbiturat-Puffer, pH 8,6, bestimmt.

Der isoelektrische Punkt wurde mit einer Säule (440 ml) der Firma LKB, Stockholm, ermittelt. Das Ampholin®-Gemisch hatte einen pH-Bereich von 5,0 bis 8,0.

Der Sedimentationskoeffizient wurde in einer analytischen Ultrazentrifuge der Firma Beckman (Spinco-Apparatur, Modell E) bei 60.000 UpM in Doppelsektorzellen mit Hilfe der UV-Scanner-Technik bei 280 nm bestimmt. Als Lösungsmittel diente Wasser. Die Proteinkonzentration betrug 2 g/l.

Zur Bestimmung des Molekulargewichts in der Ultrazentrifuge wurde die Sedimentationsgleichgewichtsmethode herangezogen. Die Konzentration des Proteins war dazu auf etwa 1,0 O.D. (optische Dichte) eingestellt worden. Als Lösungsmittel diente ein 0,05 mol/l Phosphatpuffer (pH 6,8) der 0,2 mol/l NaCl enthielt. Die Bestimmung wurde bei 9 000 UpM vorgenommen. Die Registrierung erfolgte bei 280 nm unter Einsatz eines photoelektrischen Scanners.

Die Kohlenhydrate wurden wie folgt bestimmt:
Nach Hydrolyse der glykosidischen Bindungen wurden die freigesetzten Neutralzucker als Boratkomplexe über eine Anionenaustauschersäule getrennt (Y.C. Lee et al., Anal. Biochem. 27 (1969), 567), im Eluat durch Zumischen von Cu(I)-bicinchoninatreagenz (K. Mopper und M. Gindler, Anal. Biochem. 56 (1973), 440) angefärbt und unter Verwendung von Rhamnose als internem Standard quantitativ bestimmt. Die Aminozucker wurden durch ihre Reaktion mit Ninhydrin nachgewiesen und bestimmt. Der Neuraminsäuregehalt ist mit der Methode nach Warren (Methods in Enzymology, Vol. VI (1963), 463-465) ermittelt worden.

Die Aminosäurenanalyse wurde nach S. Moore, D.H. Spackman, W.H. Stein, Anal. Chem. 30 (1958), 1185, unter Verwendung eines Flüssigkeitschromatographen Multichrom B der Firma Beckman durchgeführt. Cystin wurde nach Oxydation des Proteins mit Perameisensäure (S. Moore et al., Anal. Chem. 30 (1958), 1185) und nachfolgender Chromatographie (S. Moore, J. Biol. Chem. 238 (1963), 235) als Cysteinsäure bestimmt. Der Tryptophangehalt ist mit der direkten photometrischen Bestimmung nach H. Edelhoch, Biochemistry 6 (1967), 1948, ermittelt worden.

Bei der Untersuchung von Extrakten aus verschiedenen menschlichen Organen wurde $PP_{18}$ in relativ geringer Konzentration in Plazenta, Nebenniere und Magen mit immunchemischen Methoden nachgewiesen. Extrakte aus anderen Organen des Menschen wie Herz, Lunge, Leber, Niere, Colon, Rektum, Jejunum und Uterus enthalten dieses Protein nicht oder nur in Spuren. Mit $PP_{18}$ immunchemisch identische oder eng verwandte Proteine konnten auch in Extrakten aus Plazenten von Rhesusaffen nachgewiesen werden.

Zur Isolierung von $PP_{18}$ können demnach Organe des Menschen oder aber auch von Tieren, in denen dieses Protein vorkommt, verwendet werden. Insbesondere eignen sich dafür ausgewachsene menschliche Plazenten, da sie in reichlicher Menge anfallen und das Protein in genügend großer Konzentration enthalten.

Einer ausgewachsene menschliche Plazenta enthält im Durchschnitt etwa 2 mg $PP_{18}$.

$PP_{18}$ hat folgende Eigenschaften, die sich in einem Verfahren zu seiner Isolierung verwenden lassen, indem diesen Eigenschaften entsprechende Maßnahmen getroffen werden:
1) Mit Ammoniumsulfat wird es bei einem pH von 5-8 und 30-50% Sättigung aus wässrigen Lösungen gefällt;
2) Mit wasserlöslichen Acridinbasen, beispielsweise 2-Ethoxy-6,9-diaminoacridinlactat wird es bei einem pH-Wert von 7-9 und einer Konzentration der Base von 4 bis 8 g/l weitgehend präzipitiert. Bei einem pH-Wert von 6,0 und einer Basenkonzentration von 4 g/l fällt es nicht aus;

3) Bei Zugabe von Ethanol zu einer Lösung in einer physiologischen, oder ähnlich verdünnten, Salzlösung von pH 7 bleibt es bis zu einer Konzentration von 200 ml Alkohol pro 1 Lösung weitgehend im Überstand;

4) Bei einer elektrophoretischen Auftrennung wird es bei einem pH von 7-9 im Bereich der $\beta_1$-Globuline gefunden;

5) Bei der isoelektrischen Fokussierung erscheint es im pH-Bereich von 5,6 bis 6,2;

6) Bei einer Gelfiltration mit Sephadex® verhält es sich wie Proteine mit Molekulargewichten von 60.000 bis 100.000;

7) Es läßt sich an schwach basische Ionenaustauscher, beispielsweise DEAE-Cellulose oder DEAE-Sephadex, bei einer Leitfähigkeit von etwa 0-2 mS und einem pH-Wert von etwa pH 7 bis 9 binden und kann mit stärker konzentrierten Salzlösungen (10-50 g/1 NaCl-Lösungen) eluiert werden;

8) Es kann aus einer wässrigen Lösung durch Immunadsorption angereichert und isoliert werden.

Gegenstand der Erfindung ist demnach auch ein Verfahren zur Gewinnung oder Anreicherung des $PP_{18}$, dadurch gekennzeichnet, daß ein aus Organen, die dieses Protein enthalten, mit verdünnten Salz- oder Pufferlösungen gewonnener Extrakt einer oder mehreren der folgenden Maßnahmen unterworfen wird:

a) Fällung des Proteins $PP_{18}$ mit Ammoniumsulfat im pH-Bereich von 5 bis 8 und bei 30-50% Sättigung;

b) Abscheidung von Begleitproteinen mit einer wasserlöslichen Acridinbase bei einem pH-Wert von 6 und einer Konzentration der Base von 4 g/1 oder weniger oder Fällung des Proteins $PP_{18}$ mit einer wasserlöslichen Acridinbase bei einem pH-Wert zwischen 7 und 9 und einer Konzentration der Base 4 bis 8 g/1;

c) Abscheidung eines Teils der Begleitproteine durch Zugabe von Ethanol bei pH 7 bis zu einer Endkonzentration von 200 ml/1 Alkohol;

- 6 -                                    0165476

d) präparative Zonenelektrophorese, wobei die Proteinfraktion im Bereich der $\beta_1$-Globuline gewonnen wird;

e) Gelfiltration oder Ultrafiltration, wobei Proteine im
   Molekulargewichtsbereich von 60.000 bis 100.000 gewonnen werden;

f) Adsorption an einem schwach basischen Ionenaustauscher und Elution des Proteins $PP_{18}$;

g) immunadsorptive Anreicherung.

Neben Ammoniumsulfat können auch andere in der präparativen Biochemie üblicherweise eingesetzte Neutralsalze
zur Ausfällung des $PP_{18}$ verwendet werden. Neben einer
Acridinbase ist auch ein wasserlösliches Derivat einer
Chinolinbase, wie sie für Proteinfraktionierungen bekannt sind, im Rahmen des erfindungsgemäßen Verfahrens
einsetzbar. Entsprechend seinem elektrophoretischen Verhalten, seiner Ladung wie seinem Molekulargewicht sind
zur Isolierung des Proteins auch andere Maßnahmen brauchbar, die geeignet sind, ein Protein mit den angegebenen
Eigenschaften von anderen Proteinen abzutrennen.

Hierzu können die verschiedenen Methoden der präparativen Elektrophorese, der isoelektrischen Fokussierung,
der Gelfiltration oder Ultrafiltration oder auch die Eigenschaft des $PP_{18}$, an schwach basische Ionenaustauscher
gebunden und hiervon wieder eluiert werden zu können,
verwendet werden.

Insbesondere kann das $PP_{18}$ durch eine zweckmäßige Kombination der genannten Maßnahmen, die eine Anreicherung
des $PP_{18}$ oder eine Abtrennung dieses Proteins von anderen Proteinen bewirken, isoliert werden.

Das Beispiel beschreibt die Isolierung von $PP_{18}$ unter
Verwendung der Methode der Immunadsorption. Man könnte
zur Immunadsorption direkt den wässrigen Extrakt aus menschlichen Plazenten einsetzen. Da die Konzentration von $PP_{18}$ im Extrakt relativ gering ist, ist es zweckmäßig, durch eine Vorfraktionierung des Extraktes das Protein $PP_{18}$ erst spezifisch anzureichern.

Die im Beispiel angegebenen Schritte zur Anreicherung sind aber nicht zwingend und müssen auch keinesfalls in der dort beschriebenen Reihenfolge durchgeführt werden.

Auch der Schritt der Immunadsorption könnte durch Anwendung anderer Trennmethoden, zum Beispiel durch präparative Elektrophorese und isoelektrische Fokussierung ersetzt werden.

Zum Nachweis und zur Bestimmung des $PP_{18}$, etwa in einer Fraktion aus einer Trennoperation, können neben den angegebenen Parametern auch immunchemische Methoden dienen, da $PP_{18}$ antigene Eigenschaften hat.

Ein für diesen Zweck brauchbares Antiserum kann folgendermaßen gewonnen werden:
Bei der Fraktionierung eines Plazentenextraktes mit 2-Ethoxy-6,9-Diaminoacridinlactat und Ammoniumsulfat nach Bohn, H., (Arch.Gynäkol. (1971) 210, 440) geht $PP_{18}$ zur Hauptsache in die Plazentafraktion III. Trennt man diese Fraktion weiter durch Gelfiltration an Sephadex G-150 auf, so erscheint $PP_{18}$ im Bereich der Proteine mit Molekulargewichten zwischen 60.000 und 100.000. Durch Immunisieren von Kaninchen mit dieser Fraktion erhält man ein polyvalentes Antiserum, das unter anderem Antikörper gegen $PP_{18}$ enthält. Dieses Antiserum kann durch Absorption mit normalem menschlichen Serum und mit Plazentafraktionen, die $PP_{18}$ nicht enthalten, gegen das Antigen $PP_{18}$ weitgehend spezifisch gemacht werden.

Dieses Antiserum kann einerseits zum immunologischen Nachweis des $PP_{18}$, andererseits zur Herstellung eines Immunadsorbens dienen, das zur Anreicherung und Isolierung von $PP_{18}$ eingesetzt werden kann.

Mit Hilfe des nach dem Beispiel der vorliegenden Anmeldung gewonnenen gereinigten $PP_{18}$ lassen sich durch Immunisieren von Tieren nach bekannten Methoden monospezifische Antiseren herstellen.

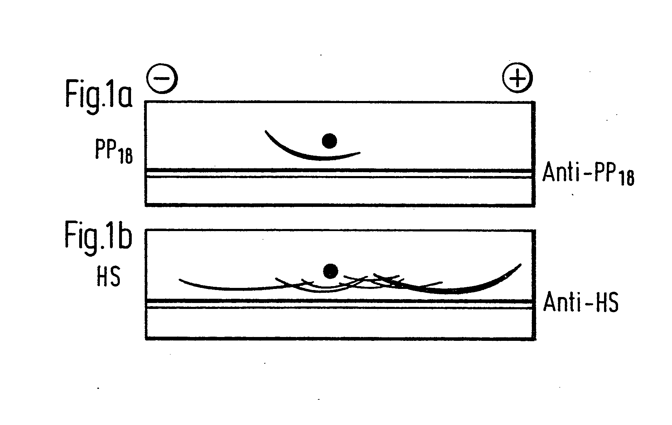

Figur 1a zeigt die immunologische Reaktion von $PP_{18}$ mit einem spezifischen Antiserum vom Kaninchen nach Auftrennung im elektrischen Feld in Agar-haltigem Gel.

Figur 1b bringt zum Vergleich dazu die Auftrennung der Proteine des Serums, sichtbar gemacht durch deren Immunreaktion mit einem Antiserum vom Kaninchen gegen Humanserum (HS).

Zum immunologischen Nachweis von $PP_{18}$ können auch die Geldiffusionstechnik nach Ouchterlony (Schultze and Heremans, Molecular Biology of Human Proteins, Vol. 1, pg. 134) oder, wenn notwendig, auch empfindlichere Methoden wie Radioimmunoassays oder Enzymimmunoassays herangezogen werden.

Der Nachweis und die Bestimmung von $PP_{18}$ haben diagnostische Bedeutung. $PP_{18}$ ist ein Gewebeprotein, das nur in bestimmten Organen in größerer Konzentration vorkommt. Bei einer Erkrankung dieser Organe kann infolge eines vermehrten Zellzerfalls die Konzentration des Gewebeproteins $PP_{18}$ im Serum oder in anderen Körperflüssigkeiten der Patienten über die Norm ansteigen. Der Nachweis und die Bestimmung von $PP_{18}$ in Körperflüssigkeiten können daher zur Erkennung von Erkrankungen dieser Organe oder auch als Marker zur Überwachung des Krankheitsverlaufs und zur Kontrolle der Therapie eingesetzt werden.

$PP_{18}$ kann also verwendet werden, um Antiseren herzustellen, die dazu dienen können, $PP_{18}$ nachzuweisen und zu bestimmen sowie zum Aufbau von immunchemischen Methoden.

Die Erfindung wird an nachstehendem Beispiel erläutert:

<u>Beispiel</u>

A)  Extraktion der Plazenten und Fraktionierung des Ex-
    <u>traktes mit einer Acridinbase und Ammoniumsulfat</u>
    1.000 kg tiefgefrorene menschliche Plazenten wurden
    im Schneidmischer zerkleinert und mit 1.000 l einer
    4 g/l Kochsalzlösung extrahiert. Der Extrakt ist
    dann, nach Abtrennung des Geweberückstandes durch
    Zentrifugation, mit 200 ml/l Essigsäurelösung auf
    pH 6,0 eingestellt und unter Rühren mit 200 l einer
    30 g/l Lösung von 2-Ethoxy-6,9-Diaminoacridin-Lactat
    (Hoechst AG) versetzt worden. Der Niederschlag wurde
    abzentrifugiert und verworfen.   Der Überstand wurde
    mit 10 g/l Betonit A (Fa. Erbslöh & Co., Geisenheim/
    Rhein) versetzt, durch Zugabe von 2 N NaOH auf pH
    7,0 eingestellt und filtriert. Das Filtrat ist unter
    Rühren langsam mit 300 g/l Ammoniumsulfat versetzt
    worden; das Plazentaprotein $PP_{18}$ fiel dabei, zusam-
    men mit anderen Proteinen, aus. Der Niederschlag
    wurde abfiltriert. Es wurden etwa 12 kg einer feuchten Paste erhalten, die im nachfolgenden als Fraktion A bezeichnet wird.

B) <u>Fraktionierung mit Ethanol</u>
   500 g der Fraktion A wurden in 400 ml Wasser gelöst
   und bei 4°C gegen physiologische Kochsalzlösung dialysiert. Nach der Dialyse wurde die Leitfähigkeit der Lösung durch Zugabe von einer 50 g/l NaCl-Lösung auf 15 mS eingestellt. Die Lösung ist dann auf 0°C abgekühlt und unter Rühren langsam mit einem 960 g/l enthaltenden Ethanol bis zu einer Endkonzentration des Alkohols von 200 g/l versetzt worden. Der Niederschlag wurde durch Zentrifugation entfernt. Der Überstand ist zunächst gegen Wasser, dann gegen einen 0,1 mol/l Tris-HCl-Puffer (pH 8,0), der 1 mol/l NaCl und 1 g/l $NaN_3$ enthielt (Pufferlösung II), dialysiert worden. Anschließend wurden die Proteine durch Zugabe von 380 g/l festem Ammoniumsulfat ausgefällt. Der Niederschlag wurde in Wasser gelöst und gegen Pufferlösung II dialysiert. Erhalten wurden etwa 1000 ml einer Lösung (Fraktion B), die im Durchschnitt 160 mg $PP_{18}$ enthielt.

C) Anreicherung von $PP_{18}$ durch Immunadsorption

1. Herstellung des Immunadsorbens

300 ml eines Anti-$PP_{18}$-Serums vom Kaninchen wurden gegen 0,02 mol/l Phosphatpuffer (pH 7,0) dialysiert und zur Abtrennung der Immunglobuline an DEAE-Cellulose chromatographiert. Die Immunglobuline wandern dabei ungehindert durch die DEAE-Cellulose hindurch, während die übrigen Serumproteine großenteils an die DEAE-Cellulose adsorbiert werden. Die Immunglobulinfraktion (2,62 g Protein) im Durchlauf wurde dann mit 262 g besonders gereinigter Agarose in Kugelform (Sepharose® 4 B der Pharmacia, Uppsala, Schweden), die mit 32,7 g Bromcyan aktiviert worden war, umgesetzt und so kovalent an einen Träger gebunden. Ein geeignetes Verfahren ist beispielsweise beschrieben von Axen el al., Nature 214, 1302 (1967).

Mit Hilfe eines auf diese Weise hergestellten Immunadsorbens konnte das Protein $PP_{18}$ aus seiner Lösung, insbesondere aus $PP_{18}$ angereicherten Plazentenfaktionen, isoliert werden.

## 2. Durchführung der Immunadsorption
----------------------------------

Das Immunadsorbens wurde in Pufferlösung II (0,1 mol/l Tris-HCl-Puffer, pH 8,0, mit 1,0 mol/l NaCl und 1 g/l $NaN_3$) suspendiert, in eine Chromatographiesäule gefüllt (5,0 x 15 cm) und mit Pufferlösung II nachgespült. Dann wurde die halbe Menge der Fraktion B auf die Säule aufgetragen, wobei $PP_{18}$ immunadsorptiv gebunden wurde. Die Säule wurde gründlich mit Puffer II gespült. Anschliessend ist das adsorbierte Protein mit etwa 600 ml 3 mol/l Kaliumrhodanid-Lösung von der Säule eluiert worden. Die $PP_{18}$-haltigen Eluate wurden gegen Pufferlösung II dialysiert und im Ultrafilter auf etwa 10 ml eingeengt. Ausbeute pro Adsorption etwa 6 mg $PP_{18}$.

Das Adsorbens in der Säule wurde unmittelbar nach der Elution von $PP_{18}$ wieder mit Pufferlösung I neutralisiert und gründlich gewaschen. Es ist dann erneut zur immunadsorptiven Bindung von $PP_{18}$ eingesetzt worden.

## D) Hochreinigung von $PP_{18}$
_____

Das durch Immunadsorption gewonnene Protein war häufig noch durch unspezifisch gebundene Serumproteine und andere Plazentaproteine verunreinigt. Die Abtrennung der Hauptmenge der Begleitproteine gelang durch Gelfiltration an Ultrogel® AcA 34 und anschließende Ionenaustauschchromatographie an DEAE-Sephadex. Die Elution der an DEAE-Sephadex adsorbierten Proteine erfolgte mit einem linearen Salzgradienten von 0 bis 20 g/l Kochsalz in 0,01 Mol/l Tris-HCl Puffer (pH 7,0). Der Nachweis von $PP_{18}$ in den Fraktionen wurde mit immunchemischen Methoden durchgeführt. Die Fraktionen, welche die Hauptmenge $PP_{18}$ enthalten, wurden vereinigt und auf einem Ultrafilter eingeengt. Die restlichen Begleitproteine wurden dann durch inverse oder negative Immunadsorption, das heißt mit Hilfe von trägergebundenen Antikörpern gegen die noch als Verunreinigung vorliegenden Proteine, entfernt. Zur Herstellung von Antikörpern gegen diese Begleitproteine wurde ein Teil der $PP_{18}$-Rohfraktion zwölf Stunden gegen einen 0,5 M Glycin-HCl-Puffer (pH 2,5) dialysiert und dann wieder neutralisiert. Unter diesen Bedingungen wird $PP_{18}$ denaturiert und verliert seine immunchemische Reaktivität; die Verunreinigungen dagegen sind stabil und bleiben als Antigen erhalten. Immunisiert man damit Tiere, so erhält man Antikörper gegen diese Proteine, die nach Bindung an einen Träger zur immunadsorptiven Entfernung der unbekannten Plazentaproteine aus der $PP_{18}$-Rohfraktion eingesetzt werden können.

Patentansprüche:

1. Protein $PP_{18}$, gekennzeichnet durch

   a) eine elektrophoretische Beweglichkeit im Bereich
      der von $ß_1$-Globulinen;

   b) einen isoelektrischen Punkt zwischen 5,6 und 6,2;

   c) einen Sedimentationskoeffizienten $s_{20,w}$ von $5,0 \pm$
      $0,2$ S;

   d) ein in einer Ultrazentrifuge bestimmtes Molekular-
      gewicht von $82\ 300 \pm 5\ 600$; und

   e) einen Kohlenhydratanteil von $2,3 \pm 1,3$ g/100 g
      (Mannose $0,15 \pm 0,1$, Xylose(?) $0,5 \pm 0,5$, Galactose
      $0,5 \pm 0,2$, Glukose $0,2 \pm 0,1$, N-Acetyl-Glukosamin
      $0,7 \pm 0,2$, N-Acetyl-Neuraminsäure $0,25 \pm 0,2$, je-
      weils g/100 g).

2. Verfahren zur Gewinnung oder Anreicherung des $PP_{18}$
   nach Anspruch 1, dadurch gekennzeichnet, daß ein aus
   Organen, die dieses Protein enthalten, mit verdünnten
   Salz- oder Pufferlösungen gewonnener Extrakt einer
   oder mehreren der folgenden Maßnahmen unterworfen
   wird:

   a) Fällung des Proteins $PP_{18}$ mit Ammoniumsulfat im
      pH-Bereich von 5 bis 8 und bei 30-50% Sättigung;

   b) Abscheidung von Begleitproteinen mit einer wasserlöslichen Acridinbase bei einem pH-Wert von 6 und
      einer Konzentration der Base von 4 g/l oder weniger
      oder Fällung des Proteins $PP_{18}$ mit einer wasserlös-
      lichen Acridinbase bei einem pH-Wert zwischen 7 und
      9 und einer Konzentration der Base 4 bis 8 g/l;

   c) Abscheidung eines Teils der Begleitproteine durch
      Zugabe von Ethanol bei pH 7 bis zu einer Endkon-
      zentration von 200 ml/l Alkohol;

   d) präparative Zonenelektrophorese, wobei die Protein-
      fraktion im Bereich der $ß_1$-Globuline gewonnen wird;

   e) Gelfiltration oder Ultrafiltration, wobei Proteine im Molekulargewichtsbereich von 60.000 bis 100.000 gewonnen werden;

f) Adsorption an einem schwach basischen Ionenaustauscher und Elution des Proteins $PP_{18}$;

g) immunadsorptive Anreicherung.

3. Verwendung des Proteins $PP_{18}$ nach Anspruch 1 zur Gewinnung eines Antiserums und zum Aufbau von immunchemischen Methoden zum Nachweis und zur Bestimmmung dieses Proteins, zur Erkennung von Erkrankungen, zur Überwachung von Erkrankungen oder zur Kontrolle einer Therapie.

0165476

Fig.1a

Fig.1b